# EUROPEAN PATENT SPECIFICATION

(11) **EP 2 488 067 B1**
(45) Date of publication and mention of the grant of the patent: **25.12.2013**
(21) Application number: 10752616.2
(22) Date of filing: 19.07.2010
(51) Int. Cl.: A47G 9/00, A47C 31/00

(54) **MULTILAYERED PROTECTIVE AND STIMULATING PAD FOR MATTRESSES**
MEHRSCHICHTIGES SCHÜTZENDES UND STIMULIERENDES POLSTER FÜR MATRATZEN
NATTE MULTICOUCHE DE PROTECTION ET STIMULATION POUR MATELAS

(30) Priority: 14.10.2009 HR 20090549
(43) Date of publication of application: 22.08.2012
(73) Proprietor: Pavletic D.O.O., 51000 Rijeka (HR)
(72) Inventor: PAVLETIC, Marin, 51226 Hreljin (HR)
(74) Representative: Mrdeza, Zeljka
(86) International application number: PCT/HR2010/000024
(87) International publication number: WO 2011/045622

(56) References cited:
- EP-A2- 0 963 138
- DE-U1- 29 924 926
- DE-U1-202007 001 570
- JP-A- 2008 259 592
- US-A- 5 889 923

## Description

### INVENTION DESCRIPTION

### 1. TECHNICAL FIELD

This invention refers to the multilayered protective and stimulating pad for mattresses, used as protection and stimulator of those areas of the human body where the malfunctions of the energetic earth electromagnetic field and technical radiation, harmful for the human health are present.

This invention belongs to the A area - everyday life needs and to the H area - electrical engineering, particularly to the area of circuitries, aimed for use that will have impact on the human beings, with no electric voltage.

According to the international classification, this invention could be classified as:
- A 47 C 27/00: Filled mattresses
- A 61 N 1/16: Protection or neutralising undesirable influences from atmospheric or terrestrial radiation or fields
- H 05 F 7/00: Using the electricity of natural origin

### 2. TECHNICAL PROBLEM

Harmful radiation coming from the earth and environment is known a long time hence. People long time ago become aware that areas unstable from the energetic point of view i.e. where radiesthesist can use their forked branches, plumb-line and other tools are not healthy areas to live in. Various literatures elaborates harmful impacts of the natural radiation, underground waters, Hartman and Curry network, geological clefts, various minerologic radiations, from the underground, radiation of electrotechnical devices (TVs, PCs, transmission lines as well as various other devices).

There is a huge amount of various ores and minerals having different electrical potentials due to which, a so called piezo effect causes increased voltage and currents. A huge masses and pressure forces increase friction in the mineral deposits thus creating high charges leading to the modification of energetic field on the earth surface.

Above the underground waterflow, earth is more electrically negative than its surrounding so the charghe in the air is intensified by a positive charge.

There are various electric charges which depend upon the quantity of water present in the underground flow i.e. upon the type and quantity of mineral deposits in the underground.

It is known that disturbet energetic fields harmfully impact on all living organisms as they interfer the natural flow of biologic currents managing the living processes inside the cellular structures. Interfering biochemical and bioelectrical functions of the living organisms surely leads to drooping of all organism's functions.

The essence of the technical problem which is to be solved by this invention is to eliminate impact of various electromagnetic fields and charges and reestablish the energetic balance using the multilayered protective and stimulating pad for mattresses. All mentioned is done to prevent the psychophysical health of the man and improve the quality of energy flow inside the human body.

### 3. BACKGROUND ART

Until now people protected themselves from harmful radiations in their living and working areas by using various suggestive and autosuggestive methods and various blocking devices present on the market the majority of which do not offer any possibility of measurement. It is necessary to emphasize that areas where radiation harmful for health are present usually have very low energetic values of interfered electromagnetic field so the harmful impact is based on long-term exposure of the organism to that radiation.

New scientific discoveries in the field of physics, medicine and electrical engineering have explained numerous unknown facts that caused a more quality approach against health harmful radiations.

This invention is based on the multi-layer systems of which every protection layer has a functional role.

Intention was to make this pad for mattresses which have not only the protectional function but also to be the protection and stimulative pad for mattresses.

It means that one part of the pad works as a protection system against various interfered electromagnetical earth and technical radiations whilst the other part stimulates the circulation of energies inside the human body by crystalotherapy system and beneficial effect of aromatherapy with lavender oils used.

All devices and protections known by now do not have such a great and satisfactory protection and stimulation as this pad has, all according to the invention.

In available professional literature, scientific and research papers covering various areas were elaborated problems related to the harmful radiations and their impact on organism development and life thus also on the human bioenergetic system. Multilayered protective and stimulating pad for mattresses according to the invention was made on the basis of available experiences and various professional literature: Gornik Mirko: "Bioarchitectonic basis of the space, construction and buildings", Gortan-ing d.d., Zagreb, Zagreb 1991, Jakobovic Zvonimir : "Ionizing radiation and man", 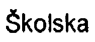 knjiga Zagreb, 1991; Perme Torno: "Structure of nature", National and University Library, Ljubljana, 1988; 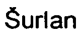 Arsen : "Biomedical engineering", Svjetlost, Sarajevo, 1978 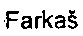 Boris: "Radiesthesia in practice", G.O. Centar, Zagreb, 1985. 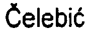 Nikola: "Bioenergy and life", Salkić, Zagreb, 1998; 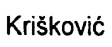 Pavao: "Homeopathy", Tiskara Rijeka, 1976, Andreas Peter-Kilian Casper: "Fantastic scientific researches", Stvamost, Zagreb, 1974; Clarke C. Arthur: "A world of mystic forces", August Cesarec, Zagreb, 1986. Perković M.- Topalović M. : "Acupuncture", 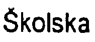 knjiga Zagreb, 1983, Lamaja 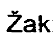 : "Medicine of residence", 1993. Translated by Vilim Kanjski: Men surrounded by harmful radiations, Rijeka Technological Centre, 1996. Matilda 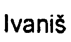 : "Aromatherapy - small home pharmacya", Planetopija d.o.o.,2006.; Chrissie Wildwood : The Bloomsbury of Aromatherapy (translated by 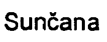 Tuksar),_Biovega Zagreb, 2002.

The applicant does not have any information about identical or similar solution.

DE 20 2007 001 570 U1 discloses a multilayered protective and stimulating pad according to the preamble of claim 1.

All solutions known till now have their disadvantages compared with here proposed solution, according to invention. Those disadvantages refer to the complexity of device design as well as at the very narrow area of usage. Multilayered protective and stimulating pad for mattresses according to the invention has both the protective function against various harmful radiations and stimulative effect on the man.

### 4. 4. DISCLOSURE OF THE INOVATION

Nature is based on circulation i.e.flow of energy that influence the living world. Those energetic flows in nature have interferences causing disturbance of the normal functioning of flora and fauna as well as disturbance of human cells functions. There are numerous causes of energetic flows disturbance.

Energetic flows disturbances are demonstrated in various forms but the most important is that man as a human being does not function well (disturbances in organism, various illnesses, tire, depression and similar).

The main purpose of this invention is to combine special electroconductive types of textile with spectral crystal layer and layer of textile soaked in aromatic oil and achieve as higher possible protective and stimulative effect on the human body.

It is achieved by the multilayered protective and stimulating pad as claimed in claim 1.

When electromagnetic streaming earth - sphere comes across the multi-layer electroconductive textiles that have net weaving, alternating streaming current that reaches them will burn inside those square nets, by a system of inducted short circuit currents (streams).

In a simple way it decreases the harmful impact of radiation on the human body. Purpose of the crystal and spectral coat situated in the pad is typical crystal and therapeutical activity, stimulating the energetic revitalization of a person lying on a multilayered pad, according to the invention, whilst evaporation of lavender essential oils by their aromatherapeutical activity ensure an undisturbed and refreshing sleep.

Technical novelty of this multilayered protective and stimulating pad for mattresses according to the invention in relation to already known solutions is that there are huge differences in design and elevated functional activity.

Its protective activity is based on decreasing the earth and technical radiations by a system of short circuit currents (streams).

Stimulative therapeutic activity is performed by crystalotherapy and aromatherapy.

Functionality of this protective and stimulating pad for mattresses is verifiable by measuring as well as by alternative shooting of energetic changes in the aura field and by increased energetic impact of energy flows inside the human body.

### 5. BRIEF DESCRIPTION OF DRAWINGS

Picture 1 shows the section of the pad according to the invention which have multiple layers

### 6. DETAILED DESCRIPTION OF INVENTION

Multilayered protective and stimulating pad for mattresses, according to the invention and as visible on the Picture 1, is made of following layers, layer of textile treated by aromatherapeutic essential oils (1) at the upper and lower part of the pad, layers of flysch (2) which are situated above and below the textile treated by aromatherapeutic essential oils, layer of textile with carbone fibres (3), layer of textile with silver fibres in a form of small squares creating a a net (4), layer of fractal stake made of crystals and minerals (5) and a layer of highly elastic memory latex foam (6).

Textile treated by aromatherapeutic essential oils (1) and made of cotton 70% and polyester 30% is, situated at the external side of the pad according to the invention. It is treated by aromatherapeutic essential oils (Lavender). Purpose of treating the textile by lavender oil is aromatherapeutic impact on the human body.

The essence of aromatherapy is treatments with aromas. Aromas used in aromatherapy have positive psychological and emotional impact that calm down and equilibrate mind, spirit and body. Aromatherapeutic essential oils that are absorbed by skin helps in equilibrating the corporal systems under stress and ensure an undisturbed and refreshing sleep.

Solution with essential lavender oil which has a strong antiseptic activity, disinfects the skin, has anti-inflammatory impact, stimulates the tissue regeneration and has a calm impact on the nervous system. Lavender essential oil is natural, useful and mild mean against restlessness, anxiety, depression and insomnia.

Using of the invention foreseen using the lavender essential oil but allows using any other aromatic oil.

Flysch (2) is situated above and below the textile treated by aromatherapeutic essential oils and ensures the mildness of the pad, according to the invention.

Textile of carbon fibres (3) is weaved by carbon fibres which have a form of small square net. Carbon fibre is a synthetic fibre of a high modulus created of the carbon atoms. It has a significantly low stretching ability and is not exposed to the UV-radiation:

Textile of carbon fibres which have a form of small square net has about 92% of textile and 8% of carbon fibres. Carbon textile electrical resistance is 4.7 KΩ/m = 4700 Ω/m

Carbon fibres textile is obtained using a high technology of inserting the carbon fibres and weaving them in a net form. It protects by forming the obstacle against electromagnetic impurance as carbon absorbs harmful radiations by transforming them in a minore heat and then release it in the air.

Weaved into mattresses and pad, carbon net ensure a quality sleep, great relaxation and a pleasant feeling every night.

Layer (4) is a textile with silver fibres weaved in the form of small squares making a net. This textile is mainly used as a basic bedlinen (blankets, pillows and coverlets). It is produced as silver light textile name. As the majority of textiles used for bedlinen have a relatively high ohm resistance, this type of textile with silver fibres has a really low resistance of 206 Ω/m which, thanks to the square weave, burning of electricity by using the inducted short circuit streams.

Regarding a low textile resistance, in the contact with the physical aura of a body and with help of a whirling streaming, release the accumulated and exaggerated electricity from our body. Thanks to such activity, nervous tension decreases and organism is calming down which consequently leads to a calm sleep.

Every material and silver also has its own resonant moleculare frequency that partially enters the area around the net weaved with silver, inside the textile. As numerous bacteria, viruses and various microorganisms inside our body or in our surrounding, do not support this silver vibrating frequency, die of it or simply avoid it completely, if possible.

This cognition confirms the fact that it is possible to perform a high quality anti-bacterial programme by using this textile.

It is certain and measurable that this textile eliminates electricity on the side on which the person is lying but make it also at the other side directed to the electrical field that is surrounding the bed. A logic conclusion that is also measurable is that a great part of the electricity will be eliminated from the surrounding area. Here we can include technical radiations of various electrical features as well as those which we call geopathogenic radiation, coming from earth.

Due to its weaving, this textile is very ethereal which means that adequate quantity of air and transpiration will be enabled in contact with a person's body so there will be no humidity in the bed area which is frequent and harmful for health.

A proof that this type of textile with weaved silver fibres has a great value was confirmed by the Zimmermann company in Bavaria, Germany, which produced a material called "eBlocker". That material does not flow through 99.999 % of electromagnetic radiation what is shown by all former instrumental measurings.

Textile with silver fibres in relation to all other textiles used for bedlinen is useful in many ways for prevention and healing the human health thanks to the aforementioned features. Combining it with other materials of natural origin, we get more efficient and useful features of bedlinen from the health point of view.

Finally, silver is used in medicine for decades and recently researches showed that silver fibre that is combined with textile in production of mattresses and pads is particularly favourable for people who want to protect themselves against microbes. In production of mattresses and pads, silver fibre is irrecoverably connecting with polymers so becoming physically unseparable which guarantee a long antimicrobial function.

Scientists have proven that silver has great antibacterial features and was also used for healing wounds. Today, thanks to the technological improvements, silver fibre is attaching to the fibre itself thus having an antimicrobial impact.

Textile weaved by silver fibres directly slather electrostatic charge as metal is a great conductor and capable of distributing uniformly the body temperature. Silver fibres prevents multiplication of bacteria, fungi and bad fragances. Due to its electrical conductivity, it has antistatic impact.

Fractal stake (5) is nothing else but fractal of a mineral and crystal structure of negative polarization. Fractal stake as a whole oscillates from 10Hz to 900Hz. Crystals, minerals and precious metals (Green Tourmaline, Quartz, Tourmaline Flint, Calcite etc. and gold petals in traces) from the stake, according to invention, balance and strenhgten the body energy and regenerate the bioenergetical field status. Thus their improve the general health status and at the same time protect efficiently against natural and technical radiations. By radiating vibrations crystals encourage human's natural capacity of self-healing. Their vibrations are very harmonious, they send off the energetic warmness and impact on some human organs. Crystals and minerals in the stake, according to the invention, balance and strenhgten the body energy and regenerate the bioenergetical field status. Thus their improve the general health status and at the same time protect efficiently against natural and technical radiations. When human energies are equilibrated, it is manifested in the physical world as health.

Positive impacts of crystals to the human health are well known since long time. They are helpful in health difficulties and health protection and are support in healing. Crystalotherapy for years use crystals and minerals for healing. They have their own intelligence which is showed in radiating steady vibrations in predetermined time intervals thus they are part of medicine laser chips and quartz clocks. Crystals (e.g. quartz) is the basic material for PC's microprocessors production. Its task is to memorize all data saved into it. Vibration occurs due to electron discharge from the external atom shell. As crystals get powered from energetically strong earth areas, building-in such crystals into the mattress it becomes energetically good area.

Highly elastic memory latex foam (6) has a great density and open cells. One of the basic features of the memory foam is that its extensibility is very small which enable it to get a form of a body to support. In that way it supports the backbone, getting the form of the body thus enabling the adequate development of children skeletal system and diminishes the risk of skelet malformations in adult stage. Besides that, it has a good anti-alergical level thanks to a constant and effective air streaming and regulates perspiration during the sleep.

Thanks to the latex which has negative polarization of their adaptive capabilities, this material is ideal for armchairs and mattresses.

Thanks to the memory foam inside the mattress, circulation is improved and need to find a most adequate place during the sleep decreases. This also improves the quality of sleeping.

Every pad layer, according to the invention, has their basic functional value. So, carbon fibre eliminates electrostatic charge, silver fibre eliminates electromagnetic radiation, crystal fractal stake has a crystalotherapeutic impact, textile treated by aromatherapeutic essential oils has aromatherapeutic impact, and highly elastic memory latex foam ensures negative polarisation, comfort and clarity whilst flysch guarantee the mildness of the pad. All these layers make a whole and a complete functional complex. Totality of the functional values of the multilayer composition of the pad fulfill all requirements for the construction of this protective and stimulating pad, all according to the invention.

Examination of effectiveness of the multilayer protective and stimulating pad for mattresses, according to the invention, performed by GDV Kirilian camera on different people showed mainly the positive impact on people. Extremely positive changes were obtained in the liver and throat area and better aura deployment. It is necessary to taking in consideration that testing was very short (1 hour) so major changes in density of the aura field are not to be expected. Also should be taking into consideration different levels of sensibility of people which means that somebody needs more and somebody less hours of lying to feel the activity of pad according to the invention.

Testings of effectiveness of the pad, according to the invention, by measuring the radiation of the electrical field ELF, electrostatic field DC, magnetic field ELF and geomagnetic field DC also indicates on positive impact of the pad. Following measuring instruments were used - Metex M3850D digital voltmeter of RMS value, Spectran F-5035 digital analyser of the electric field, digital magnetometer Geo-scaner BPT 3010, compass M-73 type and digital measuring device of the ELF magnetic field EMF-822A.

The area for measuring the electrical field ELF was realized on a horizontal glass surface some 80 cm above the floor. The following results were obtained during measurement:
2. With no voltage 230 V, 50 Hz, without connecting pad to the earthing: up 20 cm 5V/m and down 20 cm 2,1 V/m, directly on the pad up 0,6 V/m. With the pad connected to earthing: up 20 cm 5,5 V/m and down 6,5 V/m directly on the pad up 0,9 V/m.
3. With voltage present 230 V, 50 Hz some 40 cm above the floor, without connecting pad to the earthing: up 20 cm 10,3 V/m and down 20 cm 350 V/m, directly on the pad up 1 V/m. With the pad connected to earthing: up 20 cm 6,9 V/m and down 370 V/m directly on the pad up 1,2 V/m.

| Pad electrical field | No earthing | With earthing |
|---|---|---|
| Above the pad V/m | 10,3 | 6,9 |
| Below the pad V/m | 350 | 370 |
| Value of the field on the pad (V/m)% | 2,9% | 1,8% |
| Pad efficiency (V/m)% | 97,1% | 98,2% |

Measuring area of the pad according to the invention surrounded by a high level of the electric field - 350 V/m below the pad, when it is not connected to earthing, significally impact on weakening the electric field above the pad to 10,3 V/m (or approx. 2,9% of the level measured at the lower side), efficiency of the pad according to the invention is 97,1 %.

Measuring area of the pad according to the invention, surrounded by a high level of the electric field - 370 V/m below the pad, when it is connected to earthing, significally impact on weakening the electric field above the pad to 6,9 V/m (or approx. 1,8% of the level measured at the lower side), efficiency of the pad according to the invention is 98,2%.

As pad was produced of passive electrostatic materials and has a textile with net of antistatic material, creation of the electrostatic potential is not registered.

Measuring area of the pad, according to the invention, is particularly favourable for electrostatic discharge.

Pad does not contain permanent magnets and ferromagnetic materials and do not impact on the free spreading of the geomagnetic field.

Measuring area of the pad, according to the invention, surrounded by a normal level of the geomagnetic field under the pad, when it is or it is not connected to protective earthing; do not impact on weaking of the magnetic field above the pad according to the invention.

Testings of pad efficiency, according to the invention, made by electro-acupunctural analysis of the body show that pad, in the very short period of time, has a good impact on organs functionality and their energetic potential, particularly when there are greater problems faced.

This pad, according to the invention, eliminates a great quantity of electromagnetical disturbances and provides with a health and stimulation. A percentage of decrease in health harmful radiations depends on strength of the electromagnetic field and treated surface.

Examination of effectiveness of pad, according to invention, by using biotensor; plumb-line wood - magnet, plumb-line metal - kamak and plumb-line wood-metal in relation to activities of the underground water flows, Hartmann's and Curry's knob as well as technical radiation also show the positive impact of the pad, according to the invention, as it moderates the harmful radiation in relation to activities of the underground water flow, with water flowing up to 15l/sec for 95% and above the 15 l/sec for 90%. As regards to the harmful radiations of the Hartmann's and Curry's knob and technical radiation, this pad, according to the invention, moderates harmful radiation up to 78%.

By a bioenergetic testing of a pad, according to invention, using the radiesthesia and teleradiesthesia method and specific analysis of the energetic field of the human being, a positive impact was determined. Bioenergetic field of the examinee which was disturbed by sleeping near harmful radiations was re-equilibrated as well as its general health condition. Energetic centres were reactivated up to 90% in relation to the initial state. Improvement of the metabolism exhausted by sleeping at the place with harmful radiaton present was detected. It results by a significant improvement of the general health state of the examinee by using the mattress pad according to the invention. Using the pad for mattresses according to the invention permanently, general healt condition is improved up to 95%.

Pad for mattresses is, according to the invention, described as functional designed whole with technical and measured data which explain also its effects.

Technical characteristics of the pad, according to the invention, burns the greater part of the voltage oscillations of the earth radiation. It also provides with the energetic revitalisation and is also used as antibacterial and antistress programme system for relaxation and undisturbed sleep.

Pad for mattresses according to the invention is designed as eliminator of the energetic, earth and technical radiation of the electromagnetic field that is harmful for health as well as the energetic revitalisation device and antistressing stimulator.

### 7. INDUSTRIAL APPLICABILITY

The application is visible from description and enclosed picture.

According to aforementioned characteristics, this invention represents a permanently useful system used on beds and in men's environment where interferences of the energetic field of earth electromagnetic radiation are present.

Multilayered protective and stimulating pad can be used for mattresses, pillows, and pillows for seats and similar.

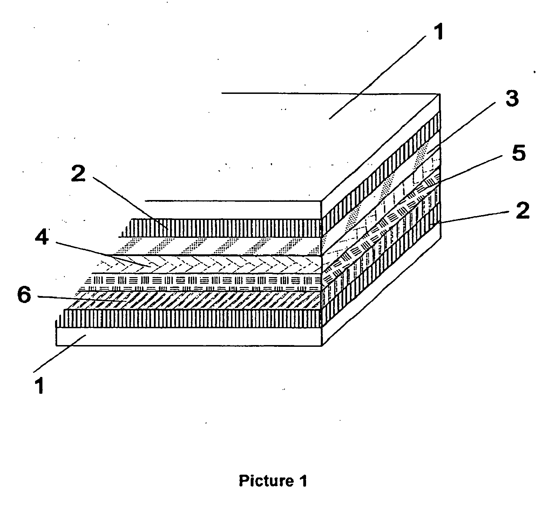

## Claims

1. Multilayered protective and stimulating pad for mattresses, being made of multiple layers, comprising a layer of textile treated by aromatherapeutic essential oils (1) at the top of the pad and a layer of highly elastic memory latex foam (6), characterized that it comprises another layer of textile treated by aromatherapeutic essential oils (1) at the bottom of the pad, layers of flysch (2) which are situated one flysch layer above the bottom layer of textile treated by aromatherapeutic essential oils (1) and another flysh layer below the top layer of textite treated by aromatherapeutic essential oils (1), layer of textile with carbone fibres (3), layer of textile with silver fibres in a form of small squares creating a net (4), layer of fractal stake made of crystals and minerals (5).

2. Multilayered protective and stimulating pad for mattresses, according to the claim 1, characterized that, the textile with carbon fibres has ohm resistance of 4700 Ω/m.

3. Multilayered protective and stimulating pad for mattresses, according to the claim 1, characterized that, the textile with silver fibres has ohm resistance of 206 Ω/m.

4. Multilayered protective and stimulating pad for mattresses, according to the claim 1, characterized that, fractal stake (5) is in fact a fractal of mineral and crystal structure, negative polarization, which oscillates as a whole from 10Hz do 900Hz.

5. Multilayered protective and stimulating pad for mattresses, according to the claim 4, characterized that, that crystals, minerals and precious metals, green tourmaline, quartz, tourmaline flint, calcite and gold petals in traces from the stake, according to invention, balance and strenhgten the body energy and regenerate the bioenergetical field status.

## Patentansprüche

1. Mehrschichtiges schützendes und stimulierendes Polster, das von mehreren Schichten hergestellt ist, enthaltend eine Textilschicht auf dem oberen Teil des Polsters, die mit den aromatherapeutischen essentiellen Ölen behandelt wurde (1) und eine Schicht des hochelastischen "memory latex" Schaums, das wiederum Flyschschichten (2) enthält, wobei eine Flyschschicht uber die untere, mit den aromatherapeutischen essentiellen Ölen behandelten Textilschicht angeordnet ist, und eine andere Flyschschicht, die unter der oberen, mit den aromatherapeutischen essentiellen Ölen behandelten Textilschicht angeordnet ist, **dadurch gekennzeichnet, daß** eine Textilschicht mit den Kohlenstofffaden (3), eine Textilschicht mit den Silberfaden in der Form der kleinen, ein Netz bildenden Quadrate (4) und eine Schicht des von Kristallen und Mineralen hergestellten fraktalen Pfahls (5), umfaßt.

2. Mehrschichtiges schützendes und stimulierendes Polster für Matratzen nach Anspruch 1, **dadurch gekennzeichnet, daß** die Textilschicht mit den Kohlenstofffaden den Widerstand von 4700 Ω/m aufweist.

3. Mehrschichtiges schützendes und stimulierendes Polster für Matratzen nach Anspruch 1, **dadurch gekennzeichnet, daß** die Textilschicht mit den Silberfaden den Widerstand von 206 Ω/m aufweist.

4. Mehrschichtiges schützendes und stimulierendes Polster für Matratzen nach Anspruch 1, **dadurch gekennzeichnet, daß** der fraktale Pfahl (5) ist wirklich das Fraktal der mineralischen und kristallinischen Struktur mit der negativen Polarisation, die als Ganzes von 10 Hz bis 900 Hz oszilliert.

5. Mehrschichtiges schützendes und stimulierendes Polster für Matratzen nach Anspruch 1, **dadurch gekennzeichnet, daß** die Kristalle, die Minerale und die Edelmetalle, der grüne Turmalin, der Quartz, der turmalinische Feuerstein, der Calcit und die goldigen Blättchen spurenweise von dem Pfahl, erfindungsgemäß, die Körperenergie ausgleichen und verstärken und den Status des bioenergetischen Feldes regenerieren.

## Revendications

1. Coussin protecteur et stimulateur multicouches pour matelas, composé de plusieurs couches, dont une couche de textile traité aux huiles essentielles aromathérapeutiques (1) au sommet du cousin et d'une couche de mousse de latex à mémoire ccimportant des couches de flysch (2) située une couche de flysch au-dessus de la couche de fond de textile traité aux huiles essentielles aromathérapeutiques et une autre couche de flysch sous la couche supérieure du textile traité aux huiles essentielles aromathérapeutiques **caractérisé en ce que** une couche de textile en fibre de carbone (3), une couche de textile en fibres d'argent en formes de petits carrés faisant un filet (4) et une couche fractale faite de cristaux et minéraux (5).

2. Coussin protecteur et stimulateur multicouches pour matelas, selon la revendication 1, **caractérisé en ce que**, le textile des fibres de carbone ait une résistance de 4.700 Ω/m.

3. Coussin protecteur et stimulateur multicouches pour matelas, selon la revendication 1, **caractérisé en ce que**, le textile des fibres d'argent ait une résistance de 206 Ω/m.

4. Coussin protecteur et stimulateur multicouches pour matelas, selon la revendication 1, **caractérisé en ce que**, la fractale (5) est en fait une fractale de minéraux et une structure cristalline, de polarisation négative, qui oscille comme un tout entre 10Hz et 900 Hz.

5. Coussin protecteur et stimulateur multicouches pour matelas, selon la revendication 1, **caractérisé en ce que**, les cristaux, minéraux et métaux précieux, la tourmaline verte, le quartz, le silex de tourmaline, la calcite et les traces de pétales d'or de la substance, d'après son invention, équilibrent et renforcent l'énergie du corps et régénèrent le statut bioénergétique du sujet
